Europäisches Patentamt

(19) **European Patent Office**

**Office européen des brevets**

(11) Numéro de publication: **0 082 084**
**B1**

# (12) FASCICULE DE BREVET EUROPEEN

(45) Date de publication du fascicule du brevet:
**14.08.85**

(51) Int. Cl.⁴: **A 61 F 5/44**

(21) Numéro de dépôt: **82402297.4**

(22) Date de dépôt: **14.12.82**

(54) **Système de filtre et d'évent intégré dans des poches de drainage d'anus artificiel et poches de drainage ainsi équipées.**

(30) Priorité: **15.12.81 FR 8123444**

(43) Date de publication de la demande:
**22.06.83 Bulletin 83/25**

(45) Mention de la délivrance du brevet:
**14.08.85 Bulletin 85/33**

(84) Etats contractants désignés:
**AT BE CH DE GB IT LI NL SE**

(56) Documents cités:
**EP - A - 0 064 044**
**FR - A - 2 310 739**
**FR - A - 2 431 285**
**FR - A - 2 443 832**
**GB - A - 1 370 622**
**US - A - 4 185 630**
**US - A - 4 211 224**

(73) Titulaire: **LABORATOIRES BIOTROL S.A., 1, rue du Foin,**
**F-75140 Paris Cedex 03 (FR)**

(72) Inventeur: **Yribarren, Philippe, Lotissement**
**Eterrena 6 allée Pierre Benoit, F-64500 Saint Jean de Luz**
**(FR)**

(74) Mandataire: **Phélip, Bruno et al, c/o Cabinet Harlé &**
**Phélip 21, rue de la Rochefoucauld, F-75009 Paris (FR)**

## Description

La présente invention concerne un perfectionnement aux poches de drainage d'anus artificiel, et plus particulièrement un système original, perfectionné, de filtre et d'évent intégré dans de telles poches.

Les patients ayant subi une opération chirurgicale telle qu'une colostomie ou une iléostomie avec création d'anus artificiel sont généralement équipés de poches de recueil réalisées en matériau souple, notamment en matière plastique, composées de feuilles ou films de ce matériau imperméables aux gaz et aux odeurs, soudées entre elles sur leur périphérie et dont l'une comporte une ouverture frontale appropriée pour assurer une communication et une liaison pratiquement étanches entre l'anus artificiel et l'intérieur de la poche.

Les excrétions solides ou liquides émises et recueillies dans cette poche s'accompagnent de l'émission de gaz intestinaux; l'évacuation des gaz contenus dans la poche est nécessaire pour éviter que celle-ci gonfle excessivement; cependant le maintien d'un minimum de pression résiduelle est vivement souhaité, pour un meilleur confort du patient et surtout pour éviter que les deux films de la poche «collapsent», c'est-à-dire se collent l'un à l'autre, formant un étranglement préjudiciable à une utilisation convenable de la poche. D'autre part, certains gaz intestinaux dégagent une odeur qui est gênante pour le patient et son entourage et il s'est donc très vite avéré avantageux de munir les poches de drainage d'anus artificiel d'un filtre placé sur le trajet des gaz de telle sorte que ceux-ci doivent le traverser avant de sortir de la poche.

Les filtres actuellement utilisés sont soit fixés extérieurement ou intérieurement sur l'un des deux films de la poche avant la réalisation de celle-ci, soit rapportés sur la face externe de l'un des deux films de la poche fabriquée préalablement.

Dans l'un comme dans l'autre de ces deux cas, des variantes ont été élaborées dans le but de rendre plus efficaces de tels filtres, tout en leur conservant un volume limité. Pour certaines de ces variantes, il s'est agi de faire dévier radialement les gaz à l'intérieur de la pastille de filtre dans laquelle ils ont pénétré soit par la périphérie, soit par un trou central, ou encore de faire suivre aux gaz un parcours complexe par un jeu de déviations et de chicanes à l'intérieur de la pastille de filtre qui a la forme d'un disque ou d'un anneau.

Dans la pratique, le filtre doit toujours rester de faible épaisseur, de manière à avoir l'incidence la plus faible possible sur l'épaisseur hors tout de la poche. Il doit cependant avoir un pouvoir filtrant et une capacité d'absorption des odeurs suffisants pour assurer au porteur d'une poche munie d'un tel filtre une autonomie allant d'un minimum de quelques heures à une durée aussi longue que 12 h, 24 h et même plus si possible; s'il s'agit d'un filtre incorporé à la poche lors de la fabrication de celle-ci, il doit en tout état de cause conserver toute son efficacité aussi longtemps que l'appareillage demeure en place. Pourtant les utilisateurs réclament de plus en plus des poches avec filtre préincorporé; c'est surtout le cas chez les patients âgés, qui n'ont pas l'habileté requise pour effectuer eux-mêmes la pose et la mise en service, souvent délicates, de filtres séparés. Quel que soit le type de filtres pour poches de drainage actuellement sur le marché, ceux-ci n'ont en règle générale qu'une autonomie limitée à 12 h au maximum, le plus souvent à 6 h et même à quelques heures seulement pour les émissions de gaz particulièrement malodorants. On peut considérer ces durées de fonctionnement comme insuffisantes, notamment quand il s'agit d'appareillages vidangeables, puisque le patient est alors contraint de changer un appareillage dont la partie collectrice est pourtant encore utilisable.

La demande de brevet FR-A No 2507468 correspondant à la demande EP-A No 0068964 de la même titulaire et non publiée à la date de priorité du présent brevet concerne un système de filtre et d'évent intégré dans des poches de drainage d'anus artificiel au cours de la fabrication de celles-ci, en association avec une partie de la zone périphérique de soudure des fils de la poche, et constituant un filtre à passage direct dont la face de sortie débouche à l'extérieur de la poche et/ou à l'intérieur d'une chambre aval qui fait partie intégrante de la poche ou la complète et dont les parois peuvent être perforées pour réaliser un évent.

On a maintenant conçu et élaboré un système perfectionné de filtre et d'évent pour poches de drainage, notamment pour colostomisés et pour iléostomisés, qui constitue un perfectionnement au système décrit dans la demande EP-A No 0068964 susdite et qui, tout en restant extrêmement simple, peut être incorporé à de telles poches, procure le moyen d'ajuster la durée de vie efficace du système de façon qu'elle corresponde au moins à la durée d'utilisation prévue de la poche, et permet en outre à l'utilisateur de déterminer lui-même à convenance le débit de sortie de gaz à donner à ce système à défaut d'une préperforation pratiquée à la fabrication.

L'ensemble de ces avantages, ainsi que d'autres, ressortiront mieux de la description qui suit, où est exposée la présente invention et qui est complétée par la planche de dessin annexée, qui en illustre plus concrètement une forme de réalisation.

L'invention a pour objet un système de filtre et d'évent intégré dans une poche de drainage d'anus artificiel au cours de la fabrication de celle-ci, en association avec une partie de la zone périphérique de soudure des films formant ladite poche, ce système comportant un filtre à passage direct dont la face de sortie débouche à l'intérieur d'une chambre aval qui fait partie intégrante de la poche et dont les parois peuvent être perforées pour réaliser un évent, ledit système comportant en outre:

a) un film additionnel replié suivant une ligne de pliage et inséré entre les films de la poche en cours de fabrication et soudé avec ceux-ci de telle façon que la ligne de soudure commune rejoigne la ligne de pliure à ses deux extrémités et définisse ladite chambre aval,

b) une cartouche de matière filtrante constituant ledit filtre insérée entre les deux parties repliées du film additionnel et fixée de façon étanche aux liquides et aux gaz le long d'une fraction de ladite ligne de pliure et sur les parties correspondantes en regard du film additionnel, ainsi que sur le reste de son pourtour, à l'exception d'une zone de sortie débouchant librement dans la chambre aval, et d'une zone d'entrée débouchant dans la poche à travers une découpe ménagée dans la partie correspondante du film additionnel, ainsi que

c) des moyens de liaison étanche aux liquides et aux gaz, en une zone au moins de la chambre aval extérieure à la cartouche filtrante, entre chacune des faces externes du film additionnel et le film de la poche situé en regard, mais sans que le libre passage des gaz entre les deux parois du film additionnel soit empêché dans cette zone, pour permettre la réalisation d'un évent par perforation des films dans ladite zone.

L'invention a également pour objet une poche de drainage pour anus artificiel, dans laquelle est incorporé à demeure, en association avec une partie de la zone périphérique de soudure des films de la poche, un système de filtre et d'évent, le filtre débouchant à l'intérieur d'une chambre aval qui fait partie intégrante de la poche et l'évent étant réalisé par perforation appropriée d'au moins une des parois de la chambre aval, caractérisée en ce que le système de filtre et d'évent correspond à l'agencement décrit ci-dessus.

Pour la mise en œuvre de la présente invention, le filtre qui constitue la base du système de filtre susdit est avantageusement composé d'une cartouche filtrante parallélépipédique, cylindrique ou autre. Pour simplifier l'exposé, l'invention sera décrite ci-après en référence à une cartouche filtrante parallélépipédique, mais il est clair que toute autre forme de filtre appropriée conviendrait également, pour autant qu'elle soit compatible avec les autres éléments du système de filtre.

La matière de filtrage constituant la partie active du filtre peut être granuleuse, fibreuse ou pulvérulente; une matière convenant tout particulièrement est par exemple constituée de granulés de charbon actif ou d'une mousse à cellules ouvertes, imprégnées d'une substance efficace pour retenir les odeurs, telle que du charbon actif ou tout autre composé chimique approprié.

La cartouche de matière filtrante peut être directement emprisonnée de façon étanche aux liquides et aux gaz, par exemple au moyen d'un adhésif double face, entre les deux parois en regard du film additionnel replié et occultée sur deux de ses côtés par soudure ou par collage, par exemple par l'adhésif double face, ou par tout autre moyen de liaison entre elles des deux parois en regard du film additionnel le long desdits côtés.

En variante, la cartouche de matière filtrante peut comprendre, sur l'une ou l'autre de ses deux faces principales ou les deux, un film supplémentaire imperméable aux liquides et aux gaz, qui isole et retient la masse filtrante sur laquelle il est collé (ou fixé de manière étanche par tout autre moyen) et qui est lui-même maintenu en contact étanche avec les parois concernées du film additionnel.

Selon l'invention, la zone d'entrée de la cartouche filtrante est ménagée par découpe dans la partie correspondante du film additionnel, avantageusement par une découpe à l'emporte-pièce sur une fraction de la ligne de pliure, de préférence sur une fraction de ladite ligne relativement éloignée de la zone de sortie de ladite cartouche filtrante.

Cette zone de sortie de la cartouche de matière filtrante débouche dans la chambre aval, définie plus haut, qui constitue un volume clos défini par les parois du film additionnel, plié et soudé aux films de la poche comme indiqué.

Quant aux liaisons étanches entre chacune des faces du film additionnel replié en regard d'un film de la poche et ce dernier, sur au moins une zone prédéterminée de la chambre aval extérieure à la cartouche filtrante, elles peuvent être réalisées par tout moyen approprié, notamment par collage ou par soudage. Le soudage est le moyen préféré; dans ce cas, un moyen tout particulièrement approprié pour éviter le soudage des deux parois internes de la chambre aval sur cette même zone, ce qui empêcherait la réalisation d'un évent par perforation ultérieure de ladite zone, peut consister à apposer contre l'une au moins de ces parois internes de la chambre dans la zone concernée une pastille de matériau non soudable, tel que du papier, dont la surface doit être au moins égale à celle de la soudure prévue. Cette pastille de papier peut elle-même être fixée, par exemple collée, sur l'une des parois de la chambre; elle peut en outre être colorée ou porteuse d'une indication appropriée, pour être rendue plus aisément repérable, sur l'une ou l'autre de ses deux faces ou sur les deux.

L'utilisateur n'a plus alors qu'à pratiquer aux endroits indiqués par la présence des pastilles au moins une perforation traversante, quand et comme il le juge nécessaire, pour ouvrir un évent adapté à son cas particulier d'utilisation et de débit de gaz intestinaux.

Ce système peut composer en outre, si on le souhaite, un organe adhésif amovible et sensible à la pression, par exemple une languette adhésive, sur au moins l'un des films de la poche dans la portion de ceux-ci qui constitue la zone susdite, de façon à permettre l'obturation temporaire des perforations réalisées en dessous de cet organe adhésif, après décollement au moins partiel de celui-ci.

En variante, on peut munir ce système d'au moins une préperforation de ces zones constituant l'évent.

Dans un système de filtre conforme à la présente invention, la cartouche filtrante peut avoir une épaisseur ou un diamètre n'excédant pas 3 à 4 mm environ, tandis que sa longueur peut être quelconque, mais avantageusement supérieure à 12 mm et pouvant atteindre 50 mm ou plus. De par sa conception même, ce filtre permet une ventilation désodorisée des poches de drainage d'anus artificiel d'une durée et d'une efficacité notablement supérieures à ce que permettent les systèmes de filtre qui sont actuellement sur le marché. Ce système est aussi porteur d'autres avantages, dont certains ont été signalés plus haut et dont d'autres apparaîtront à la lecture de la description qui suit.

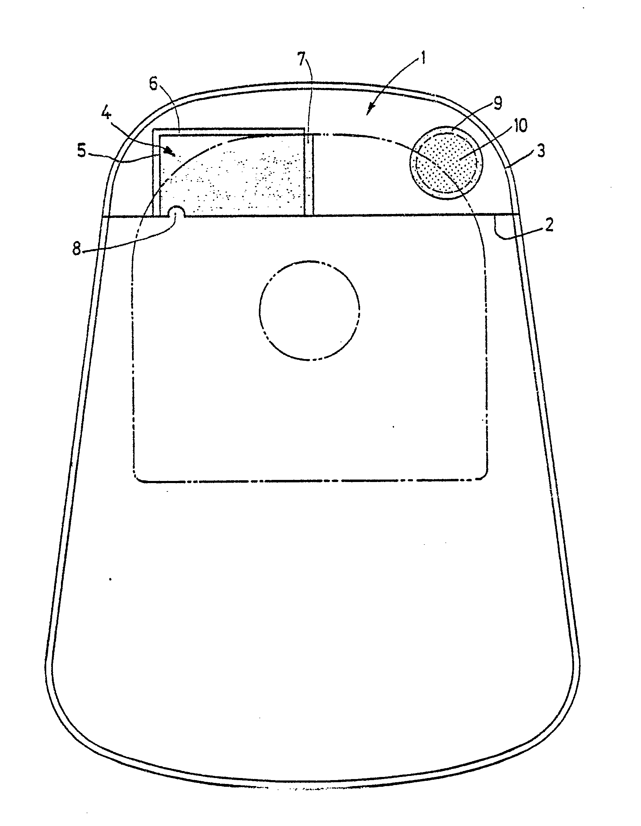

L'invention est décrite ci-après plus concrètement en référence à la figure unique ci-annexée, qui est une coupe schématique partielle d'un mode de réalisation préféré du système de filtre et d'évent intégré dans une poche de recueil conforme à la présente invention.

Cette figure annexée représente schématiquement une poche de drainage, dans laquelle se trouve disposé un système de filtre et d'évent selon l'invention comprenant un film additionnel 1 replié selon une ligne 2 et dont les deux parties libres sont soudées conjointement avec les deux films de la poche, le long d'une partie de la soudure 3 de ceux-ci. Avant la mise en place pour soudure de ce film additionnel, on a inséré entre les deux demi-films additionnels, de façon qu'elle vienne buter par un de ses côtés sur la ligne de pliure susdite 2, une cartouche filtrante 4 qu'on a ensuite fait adhérer de façon étanche, par des moyens appropriés, aux deux parois internes du film additionnel replié, l'étanchéité ainsi assurée s'étendant sur deux des côtés 5, 6 de ladite cartouche et sur les deux faces principales de celle-ci, tandis que le contact étroit contre la ligne de pliure 2 (dû au pliage ou amélioré par tout autre moyen) assure une étanchéité convenable sur un troisième côté de la cartouche également.

Outre la zone de sortie 7 ainsi dégagée, la cartouche filtrante comprend une zone d'entrée 8 qu'on a ménagée par une découpe correspondante dans le film additionnel replié, avant la mise en place de celui-ci pour soudure avec les films de la poche. Quant à la zone de perçage pour évent, elle est matérialisée par une pastille adhésive 9, colorée ou non, apposée sur l'une des parois internes du film additionnel replié avant ladite soudure, et elle est complétée par une soudure 10, à l'endroit de cette pastille et sur une surface inférieure ou au plus égale à celle de la pastille, de façon que les parois externes du film additionnel replié soient soudées sur cette zone avec la paroi interne du film de la poche en regard. Dans la pratique, cette soudure peut être réalisée avant, en même temps que, ou après la soudure des films de la poche entre eux, en une étape ou en deux. Cette soudure 10 autorise la réalisation d'un évent à la convenance de l'utilisateur, grâce à un simple perçage des films dans le périmètre de cette zone de soudure 10, qui a pour effet de laisser passer, une fois percée, uniquement les gaz contenus dans la chambre aval définie par le film additionnel 1 et qui ont traversé la cartouche filtrante 4, mais non pas les gaz non désodorisés contenus dans le reste de la poche de recueil.

**Revendications**

1. Système de filtre et d'évent intégré dans une poche de drainage d'anus artificiel au cours de la fabrication de celle-ci, en association avec une partie de la zone périphérique de soudure des films formant ladite poche, ce système comportant un filtre (4) à passage direct dont la face de sortie débouche à l'intérieur d'une chambre aval qui fait partie intégrante de la poche et dont les parois peuvent être perforées pour réaliser un évent, ledit système comportant en outre:

a) un film additionnel (1) replié suivant une ligne de pliage (2), inséré entre les films de la poche en cours de fabrication et soudé avec ceux-ci de telle façon que la ligne de soudure commune (3) rejoigne la ligne de pliure du film additionnel à ses deux extrémités et définisse ladite chambre aval,

b) une cartouche de matière filtrante constituant ledit filtre (4) insérée entre les deux parties repliées du film additionnel (1) et fixée de façon étanche aux liquides et aux gaz le long d'une fraction de ladite ligne de pliure (2) et sur les parties correspondantes en regard du film additionnel (1) ainsi que sur le reste de son pourtour (5, 6) à l'exception d'une zone de sortie (7) débouchant librement dans la chambre aval, et d'une zone d'entrée (8) débouchant dans la poche à travers une découpe dans la partie correspondante du film additionnel, ainsi que

c) des moyens de liaison étanche aux liquides et aux gaz en une zone (10) au moins de la chambre aval extérieure à la cartouche filtrante, entre chacune des faces externes du film additionnel et le film de la poche situé en regard, mais sans que le libre passage des gaz entre les deux parois du film additionnel soit empêché dans cette zone pour permettre la réalisation d'un évent par perforation des films dans cette zone.

2. Système de filtre et d'évent selon la revendication 1, caractérisé en ce que la cartouche de matière filtrante (4) est directement emprisonnée de manière étanche aux liquides et aux gaz entre les deux parois en regard du film additionnel (1) replié et est occultée sur deux de ses côtés (5, 6) par soudure, collage, ou tout autre moyen de liaison entre elles des deux parois en regard du film additionnel le long desdits côtés.

3. Système de filtre et d'évent selon la revendication 1, caractérisé en ce que la cartouche filtrante (4) comprend, sur l'un ou l'autre de ses deux faces principales ou les deux, un film supplémentaire imperméable aux liquides et aux gaz, qui isole et retient la masse filtrante sur laquelle il est fixé de manière étanche et qui est lui-même maintenu en contact étanche avec les parois concernées du film additionnel (1).

4. Système de filtre et d'évent selon l'une des revendications 1 à 3, caractérisé en ce que les moyens de liaison étanche aux liquides et aux gaz pour constitution d'un évent en une zone au moins de la chambre aval extérieure à la cartouche filtrante (4) comprennent un collage ou soudage de chacune des faces externes du film additionnel (1) avec le film de la poche situé en regard, sur une zone définissant la surface dans laquelle une préperforation existe ou un pré-perçage peut être effectué.

5. Système de filtre et d'évent selon la revendication 4, caractérisé en ce que la zone de constitution de l'évent est complétée par une pastille (9) de matériau non soudable apposée contre l'une au moins des faces internes de la chambre aval dans la zone concernée.

6. Poche de drainage pour anus artificiel, caractérisée en ce qu'elle comporte un système de filtre et d'évent selon l'une des revendications 1 à 5.

**Patentansprüche**

1. Filter- und Entlüftungssystem, das in einem Ostomiebeutel für einen künstlichen Darmausgang während der Beutelherstellung durch Verbindung mit einem Umfangszonenbereich der Verschweissung der den Beutel bildenden Folien integriert ist, wobei das System einen direkten Filterdurchgang (4) umfasst, dessen Ausgangsseite das Innere einer nachströmseitigen Kammer öffnet, die einen integralen Teil des Beutels bildet, und dessen Wände zur Verwirklichung einer Entlüftung perforiert sein können, wobei das System weiterhin umfasst
a) eine entlang einer Faltlinie (2) gefaltete, zusätzliche Folie (1), die zwischen den Beutelfolien während der Herstellung eingesetzt und mit diesen derart verschweisst ist, dass die gemeinsame Schweissnaht (3) auf die beiden Enden der Faltlinie der zusätzlichen Folie trifft und die genannte nachströmseitige Kammer ausbildet,
b) eine das genannte Filter bildende Patrone (4) aus Filtermaterial, die zwischen den beiden gefalteten Teilen der zusätzlichen Folie (1) eingesetzt und gegenüber Flüssigkeiten und Gasen längs eines Abschnittes der Faltlinie (2) und an den entsprechenden Bereichen der zusätzlichen Folie (1) wie auch an dem Rest ihres Umfanges (5, 6) mit Ausnahme einer Austrittszone (7), die die nachströmseitige Kammer freigibt, und einer Eintrittszone (8), die sich in den Beutel durch einen Schnitt in dem entsprechenden Bereich der zusätzlichen Folie öffnet, dichtend befestigt ist,
c) sowie gegenüber Flüssigkeiten und Gasen dichte Verbindungsmittel in einer Zone (10) wenigstens der genannten Kammer ausserhalb der Filterpatrone zwischen jeder äusseren Seite der zusätzlichen Folie und der betrachteten Beutelfolie, ohne dass der freie Durchgang der Gase zwischen den beiden Wänden der zusätzlichen Folie in dieser Zone verhindert ist, um die Verwirklichung einer Entlüftung durch Perforation der Folien in dieser Zone zu erlauben.
2. Filter- und Entlüftungssystem nach Anspruch 1, dadurch gekennzeichnet, dass die Filtermaterialpatrone (4) unmittelbar zwischen den beiden betrachteten Wänden der gefalteten zusätzlichen Folie (1) gegenüber Flüssigkeiten und Gasen abgedichtet gefangen ist und dass die Patrone an zwei ihrer Seiten (5, 6) durch Schweissung, Klebung oder andere Verbindungsmittel zwischen den beiden Wänden der zusätzlichen Folie längs der genannten Seiten bedeckt ist.
3. Filter- und Entlüftungssystem nach Anspruch 1, dadurch gekennzeichnet, dass die Filterpatrone (4) an der einen oder an der anderen oder an beiden ihrer Hauptseiten eine gegenüber Flüssigkeiten und Gasen undurchlässige, ergänzende Folie aufweist, die die Filtermasse isoliert und zurückhält, auf welcher sie dichtend befestigt ist, und die selbst mit den betreffenden Wänden der zusätzlichen Folie (1) in dichtem Kontakt gehalten ist.
4. Filter- und Entlüftungssystem nach einem der Ansprüche 1 bis 3, dadurch gekennzeichnet, dass die gegenüber Flüssigkeiten und Gasen dichten Verbindungsmittel zur Bildung einer Entlüftung in einer Zone wenigstens der nachströmseitigen Kammer ausserhalb der Filterpatrone (4) eine Verklebung oder eine Verschweissung jeder äusseren Seite der zusätzlichen Folie (1) mit der betrachteten Beutelfolie in einer Zone umfasst, welche die Oberfläche definiert, in welche eine Vorperforierung besteht oder eine Vorlochung durchgeführt sein kann.
5. Filter- und Entlüftungssystem nach Anspruch 4, dadurch gekennzeichnet, dass die Zone zur Bildung der Entlüftung durch ein Plättchen (9) aus nichtschweissbarem Material vervollständigt ist, das an wenigstens einer der Innenseiten der nachströmseitigen Kammer in der betreffenden Zone angebracht ist.
6. Ostomiebeutel für einen künstlichen Darmausgang, dadurch gekennzeichnet, dass er ein Filter- und Entlüftungssystem nach einem der Ansprüche 1 bis 5 aufweist.

**Claims**

1. Filter and venting device combined with an ostomy collecting pouch during the manufacture of the latter, associated to a portion of the peripheral welding area of the films forming said bag, said system comprising a straight-through filter (4) the outlet face of which opens inside a downstream chamber which is part of the pouch, and the walls of which can be perforated to produce a vent, said system further comprising:
(a) an additional film (1) folded along a fold line (2) inserted between the films of the pouch during the manufacturing process and welded with the films in such a manner that the common welding line (3) bonds the folding line of the additional film at its two ends and defines said downstream chamber,
(b) a cartridge of filtering material constituting said filter (4) inserted between the two folded portions of the additional film (1) and attached in a manner impervious to liquids and gases along a part of said folding line (2) and on the corresponding portions opposite the additional film (1) as well as on the remainder of its periphery (5, 6) with the exception of an outflow zone (7) opening freely into the downstream chamber, and an inflow zone (8) opening into the pouch through a hole in the corresponding portion of the additional film, as well as
(c) means of bonding, impervious to liquids and gases, in one area (10) at least of the downstream chamber outside the filter cartridge, between each of the outer faces of the additional film and the opposite film of the pouch, but without preventing the free circulation of gases between the two walls of the additional film in this area, to enable a venting device to be produced by perforation of the films in this zone.

2. Filter and venting device according to Claim 1, characterised in that the cartridge of filtering material (4) is directly entrapped, in a manner impervious to liquids and gases, between the two walls opposite the folded additional film (1) and is sealed on two of its sides (5, 6) by welding, sticking or any other means of bonding together the two walls opposite the additional film along said sides.

3. Filter and venting device according to Claim 1, characterised in that the filter cartridge (4) comprises, on one or other or both of its two main faces, a supplementary film impermeable to liquids and gases, which isolates and holds the filtering mass to which it is attached in an impervious manner and which is itself maintained in an impervious contact with said walls of the additional film (1).

4. Filter and venting device according to any Claim 1 to 3, characterised in that the means of bonding, in a manner impervious to liquids and gases, for forming a vent in at least one area of the downstream chamber outside the filter cartridge (4) comprise sticking or welding each of the outer faces of the additional film (1) to the opposite film of the pouch, over an area which defines the surface in which there is a pre-perforation or in which pre-perforating can be performed.

5. Filter and venting device according to Claim 4, characterised in that the area for forming the vent is completed by a patch (9) of non-weldable material affixed to at least one of the inner faces of the downstream chamber in said area.

6. Ostomy collecting pouch, characterised in that it comprises a filter and venting device according to any Claim 1 to 5.